# EUROPEAN PATENT APPLICATION

(11) **EP 4 566 589 A1**
(43) Date of publication of application: **11.06.2025**
(21) Application number: 24215950.7
(22) Date of filing: 27.11.2024
(51) Int. Cl.: A61K 9/12, A61K 9/70, A61K 33/30, A61K 47/24, A61K 47/34, A61K 47/44, A61P 17/00

(54) **PHARMACEUTICAL COMPOSITION FOR PREVENTION AND ALLEVIATION OF EARLY SKIN REACTIONS CAUSED BY IRRADIATION IN PATIENTS TREATED FOR CANCER**

(30) Priority: 27.11.2023 PL 44687223
(71) Applicant: Verco Spolka Akcyjna, 01-015 Warszawa (PL)
(72) Inventor: Jaremko-Piekarska, Emilia, 02-936 Warszawa (PL); Biegaj, Magdalena, 00-712 Warszawa (PL); Cwiklewski, Hubert, 04-035 Warszawa (PL); Kiprian, Dorota, 01-889 Warszawa (PL)
(74) Representative: JD&P Patent Attorneys Joanna Dargiewicz & Partners

(57) **Abstract**

The subject of the invention is a pharmaceutical composition for the prevention and alleviation of early skin reactions caused by irradiation in patients treated for cancer, consisting of an aqueous phase and a silicone phase, wherein the aqueous phase comprises at least one water-soluble compound as a source of zinc ions and the content of the silicone phase in the composition is not less than 10% by weight.

## Description

The object of the invention is a pharmaceutical composition for prevention and alleviation of early skin reactions caused by irradiation in patients treated for cancer.

Radiotherapy, either alone or in combination with chemotherapy, is still one of the main treatments for cancer patients. lonising radiation affects not only cancer cells but also healthy tissues within the irradiated volume. This results in radiation reactions in normal tissues. Radiation reactions affect any tissue that, due to its topography, is in the irradiated volume. Tissues that are always in the irradiated volume include the skin. Skin complications occur both after irradiation and after systemic treatment, i.e. chemotherapy or targeted treatment. The skin is made up of the epidermis and the dermis. The epidermis consists of an outer layer and a deeper layer, the so-called basal layer that is made up of stem cells with mitotic potential and the ability to differentiate into mature cells. Under normal conditions, the renewal cycle of the epidermis of the superficial layer takes about four weeks. The dermis lies beneath the epidermis and contains blood vessels, nerves, sweat and sebaceous glands and hair follicles. lonising radiation causes hydrolysis of the water inside the cell and the production of hydroxyl groups called free radicals that damage intracellular structures, particularly the DNA strand, leading to cell death by apoptosis. Cells of the basal layer that have the ability to mitigate and differentiate into mature cells are damaged. The mechanism described above is called the direct mechanism. Free radicals formed during water hydrolysis also initiate a cascade of pro-inflammatory cytokines such as interleukin-6 and 1 (IL-6,IL-1) tumour necrosis factor - TNF and interferon alpha INT- alpha that are also responsible for cell death by apoptosis. Inflammation of the epidermis is manifested not only by clinical symptoms, but also by pathomorphological changes in the cell itself. In acute radiation reactions, features of hydrotic degeneration of the basal layer of the epidermis have been described, resulting from degeneration of basal keratinocytes. There is a smoothing of the epidermal-dermal boundary, destruction of follicular epithelia and eccrine ducts with degeneration of collagen fibres in the dermis. Capillary vessels and extra-capillary venules become wide and congested, with features of endothelial cell swelling, extravasated erythrocytes and fibrin deposits around them. The lesions are accompanied by a low-grade lymphocytic inflammatory reaction of the diffuse type. Radiation complications affect only the volume of skin that, due to its topography, is located in the irradiated area. The first clinical signs of dermatitis are erythema (grade I according to the RTOG/EORT scale), followed by dry desquamation (grade II according to the RTOG/EORT scale) and moist desquamation (grade III according to the RTOG/EORT scale). Occasionally, ulcerative skin lesions develop (grade IV according to the RTOG/EORT scale). Moist desquamation phase and ulcerations (grade of reaction severity III and IV) are often accompanied by bacterial and fungal infection. All these clinical manifestations of radiation dermatitis are associated with discomfort, burning of the skin and very often also pain. The ailments described have a negative impact on patients' quality of life. Skin lesions of varying severity caused by irradiation or combined treatment with systemic therapy occur in 95% of patients. The severity of early cutaneous post-irradiation reactions depends both on the irradiation technique, the treatment regimens, i.e. combination with systemic treatment, the fractional and total dose and, to a very large extent, the individual predisposition of the patient. Late changes to the skin and subcutaneous tissue usually manifest themselves in the form of fibrosis, subcutaneous tissue atrophy and telangiectasia. Nowadays, with the use of modern irradiation techniques, increased fibrosis and telangiectasias are very rarely seen. Patients undergoing radiotherapy experience hypersensitivity of the skin to UVA and UVB radiation. This persists in varying degrees of severity until the end of life. A grade III skin reaction involves desquamation of the epidermis in a moist form. There is then a break in the epidermal continuity, which causes a risk of fungal or bacterial superinfection. In grade IV severity reaction, ulcer formation occurs. lonising radiation causes hydrolysis of the water inside the cell and the production of hydroxyl groups called free radicals. The free radicals formed during water hydrolysis initiate the production of pro-inflammatory cytokines such as interleukin 6 and 1 (IL-6, IL-1) tumour necrosis factor - TNF and INT - alpha interferon, among others that are responsible for cell death by apoptosis. Inflammation of the epidermis is manifested not only by clinical symptoms but also by pathomorphological changes in the cell itself. Given the pathomechanism of the development of cutaneous post-irradiation reactions and their nature, combination preparations containing protective ingredients that reduce inflammation and moisturise sensitive skin in the irradiated area may be effective. Zinc has antioxidant properties that help to combat the formation of free radicals. It is an important component or co-factor of more than 300 enzymes, of which superoxide dismutase, responsible for the neutralisation of the superoxide radical, is particularly important, thus making zinc a significant role in antioxidant processes. Zinc also exhibits anti-inflammatory effects. This action of Zn²⁺ ions involves the reduction of reactive oxygen species (ROS) produced by nicotinamide adenine dinucleotide phosphate (NADPH). It reduces by-products of cellular metabolism such as: - compounds containing superoxide radical (°O₂), - hydrogen peroxide (H₂O₂), - hydroxyl radicals (°OH). Silicone provides mechanical protection against skin damage and reduces transepidermal water loss from the epidermis (TEWL). The condition of human skin can be characterised, among others, by the TEWL parameter, customarily expressed in g/(m²h). This parameter depends on many factors such as age, gender, body surface area, skin temperature, perspiration or the patient's emotional state. External factors such as temperature, humidity or airflow surrounding the skin also have an influence. TEWL is a parameter characterising the barrier properties of the skin. In case of skin barrier damage, TEWL increases, which is the amount of water excreted from the body due to evaporation through the skin. Silicone leaves a protective layer on the skin, contributing to a reduction in water evaporation from the stratum corneum. By reducing TEWL, an increase in hydration of the irradiated area is achieved. Once applied, the product forms a thin protective layer that can delay the onset of post-irradiation reactions. Topical application of products with silicone has also been shown to ensure accelerated epithelialisation and reduced inflammatory response. In addition, silicone enables a moist environment to be maintained within wounds, leading to rapid healing and skin regeneration.

Publication WO2017109761 presents a composition in the form of a topical emulsion containing a combination of benzoyl peroxide, silicone compound(s) and zinc compounds (selected from zinc oxide, zinc sulphate, zinc lactate, silicone-coated zinc salts and other similar compounds) that is intended for the treatment of acne with concomitant prevention and/or treatment of scars, including acne scars, and that prevents skin problems such as dryness and irritation resulting from the use of benzoyl peroxide, and has increased durability. In its hydrophilic phase, it contains a potent active ingredient used in medicinal products: benzoyl peroxide, with widespread keratolytic, antibacterial and anti-seborrhoeic effects. The said composition is a silicone-in-water emulsion with a silicone-to-water weight ratio of about 1:0.4 to about 1:1.5. The anti-inflammatory effect of the composition is achieved by the combined action of benzoyl peroxide and zinc ions.

Publication WO2019208789 discloses a composition for skin protection comprising at least two types of linear siloxanes, wherein at least two types of linear siloxanes comprise polysiloxane and the polysiloxane content relative to the total amount of linear siloxanes is 6% by weight or more. The publication states that the composition is excellent in terms of fast drying, low irritation and drip prevention while providing water resistance. However, the composition does not exhibit antioxidant or anti-inflammatory activity.

Publication WO04096169 presents a dermatological preparation in the form of an emulsion, appearing as a cream, gel, or cosmetic milk, for topical application, intended for the prevention and treatment of specific dermatological problems such as inflammatory skin conditions. In addition to numerous active compounds in the form of plant extracts and vitamins, the composition contains zinc PCA that plays an anti-inflammatory and antioxidant role in the amount of 0.5-5% by weight. The composition further contains a high content of an aqueous phase, water-soluble rheology modifiers in the form of carbomer and xanthine gum, and traces of silicone oil, present in the amount of 0.5% by weight. The composition does not simultaneously provide mechanical protection against skin damage and does not reduce TEWL values while having anti-inflammatory and antioxidant effects.

Publication KR102513829B1 discloses a stable cosmetic composition that does not contain any preservatives and that simultaneously provides an antibacterial and anti-inflammatory effect against the skin, due to the use of a zinc compound with known antibacterial and anti-inflammatory activity. The implementation example discloses the use in the composition of a water-insoluble zinc compound: zinc oxide in the amount of 5% by weight calculated on the total weight of the cosmetic composition, and silicone in the form of a volatile silicone compound: cyclopentasiloxane, in the amount of up to 10% by weight calculated in close combination with triethyl citrate and disteardimonium hectorite. Due to the form of zinc, the composition does not exhibit antioxidant activity. The composition does not simultaneously provide mechanical protection against skin damage and does not reduce TEWL values while having anti-inflammatory and antioxidant effects.

Publication CN106109320A, on the other hand, presents a composition of a moisturising lotion in the form of an aqueous solution that prevents hyperpigmentation, counteracts skin ageing, promotes blood circulation and whitens and moisturises the skin. It contains water-soluble silicone oil in the amount of 2-9% by weight, rose essence in the amount of 11-12% by weight, distilled water in the amount of 20-34% by weight, glycerin in the amount of 2-14% by weight and zinc lactate in the amount of 2-12% by weight. The composition does not simultaneously provide mechanical protection against skin damage and does not reduce TEWL values while having anti-inflammatory and antioxidant effects. There is a product available on the market in the form of a thin breathable and self-adhesive soft skin dressing, with the trade name Mepitel Film (manufacturer: Mölnlycke Health Care AB) that can be used to prevent and alleviate the development of post-irradiation reactions in people undergoing irradiation. The dressing consists of a breathable and waterproof polyurethane film coated with soft silicone (Safetac^{®}).

Another product for direct application to post-irradiation dermatitis, damaged skin surfaces, superficial wounds and first- and second-degree burns, available under the trade name StrataXRT (https://strataxrt.com/wp-content/uploads/2022/01/Patient-Information-Leaflet-Strataxrt-2021.pdf), is a semi-occlusive and self-drying gel containing the silicones: polydimethylsiloxanes, siloxanes and alkylmethyl silicones (manufacturer: Stratpharma AG). After application and drying, the gel forms a protective layer that is both gas permeable and waterproof. The product moisturises and has a bacteriostatic effect, protecting the affected areas of the skin from infection.

In the light of a review of the current state of the art, it has not yet been possible to develop a cosmetic composition in the form of a liquid for skin care, which composition could be suitable and effective in preventing and alleviating early skin reactions caused by irradiation in patients treated for cancer, and at the same time would not contain active substances commonly used in dermatological preparations with local anti-inflammatory and antibacterial effects that are considered to be highly undesirable in the present invention, and that additionally exhibit drying or keratolytic effects. The inventor of the present invention has carried out extensive research, searching for substances effective in skin care, in the prevention and alleviation of early skin reactions caused by irradiation in patients treated for cancer.

### SUMMARY OF THE INVENTION

Unexpectedly, it has been found that the combination in a single composition of a silicone phase comprising silicones with emollient and film-forming properties, in combination with at least one compound being a source of zinc ions, for example in the form of a water-soluble zinc salt, exhibits a therapeutic and complementary effect, by preventing and alleviating early skin reactions caused by irradiation in patients treated for cancer.

Thus, the subject of the present invention is a pharmaceutical composition for prevention and alleviation of early skin reactions caused by irradiation in patients treated for cancer, consisting of an aqueous phase and a silicone phase, the aqueous phase comprises at least one water-soluble compound as a source of zinc ions and the content of the silicone phase in the composition is not less than 10% by weight.

Preferably, the water-soluble compound being the source of zinc ions is a zinc salt selected from organic or inorganic salts in the amount of 0.10 - 5.00% calculated as zinc.

Preferably, the organic salt of zinc is selected from the group comprising pyrrolidonecarboxylic acid zinc salt (zinc PCA), zinc lactate, zinc gluconate, zinc citrate and mixtures thereof.

Preferably, the claimed composition comprises a silicone oil as a component of the silicone phase. It is then more preferable that the amount of silicon oil is in the range of 10.00 - 90.00% by weight. It is also preferable that the silicone oil is selected from the group comprising dimethicones and mixtures thereof. It is further preferable that the viscosity of the silicone oil is in the range of 6 cSt - 300,000 cSt.

Preferably, the silicone phase comprises a silicone elastomer. Then, preferably, the amount of silicone elastomer is in the range of 1.00 - 60.00% by weight. It is also preferable that the silicone elastomer is selected from the group comprising dimethicone copolymer, dimethicone and vinyl dimethicone copolymer and mixtures thereof.

Preferably, the silicone phase comprises a silicone resin. It is then preferable that the silicone resin is trimethylsiloxysilicate.

Preferably, the silicone phase comprises a silicone rubber. It is then preferable that the silicone rubber is dimethiconol.

Preferably, the silicone phase comprises at least one alkylated dimethicone selected from the group comprising capryl methicone, cetyl dimethicone, stearyl dimethicone and mixtures thereof.

Preferably, the silicone phase further comprises vegetable oil as a single oil or a mixture of oils, in a total amount of 1.00 - 30.00% by weight.

Preferably, the silicone phase further comprises caprylic and capric acid triglycerides in a total amount of 1.00 - 30.00% by weight.

It is preferable that the silicone phase further comprises a volatile silicone. Then the amount of volatile silicone is preferably in the range of 5.00 - 30.00% by weight. Then also the viscosity of the volatile silicone is preferably in the range of 0.65 cSt - 8.0 cSt. Furthermore, then it is preferable that the volatile silicone is selected from: trisiloxane, disiloxane, hexamethyldisiloxane, cyclotetrasiloxane, cyclopentasiloxane, cyclohexasiloxane and mixtures thereof.

Preferably, the silicone phase comprises silicone oil alone or in combination with an ingredient selected from the group comprising silicone elastomer, silicone resin, silicone rubber, alkylated dimethicone and mixtures thereof.

Preferably, the silicone phase further comprises a silicone emulsifier.

Preferably, the aqueous phase further comprises allantoin.

Preferably, the aqueous phase further comprises dexpanthenol.

Preferably, the aqueous phase further comprises at least one polyhydric alcohol selected from the group comprising propylene glycol, glycerin, xylitol, sorbitol and mixtures thereof.

Preferably, the composition as defined above is intended for application in spray form.

### DETAILED DESCRIPTION OF THE INVENTION

The subject of the invention is shown in detail in the manufacturing example below and in the drawing in which:
Fig. 1 shows the change in patients' performance on the ECOG scale between visit 0, 1, 2, 3 and the follow-up visit after the therapy,
Fig. 2 shows the change in pruritus sensation between visit 0 and the follow-up visit, evaluated using the VAS scale,
Fig. 3 shows the change in burning sensation between visit 0 and the follow-up visit, evaluated using the VAS scale, and
Fig. 4 shows the change in general well-being between visit 0 and the follow-up visit, evaluated using the VAS scale.

The subject matter of the invention is presented in detail in the following implementation examples that are illustrative only and are not intended to limit the scope of the invention in any way.

### Example

The composition according to the invention has the following general composition:

| | **Components** | **% by weight** |
|---|---|---|
| | **Silicone phase: not less than 10%** | |
| 1 | Silicone oil with a viscosity range of 6 cSt - 300,000 cSt The preferable viscosity range for silicone oil is 20 cSt - 100,000 cSt | 10.00 - 90.00% |
| 2 | Volatile silicone with viscosity in the range of 0.65 cSt - 1.0 cSt | 5.00 - 30.00% |
| 3 | Silicone elastomer | 1.00 - 60.00% |
| 4 | Vegetable oils | 5.00 - 30.00% |
| 5 | Caprylic/Capric Acid Triglycerides | 0.10 - 30.00% |
| 6 | Silicone resin | 0.50 - 20.00% |
| 7 | Silicone rubber | 0.50 - 20.00% |
| 8 | Alkylated dimethicone | 0.10 - 30.00% |
| 9 | Silicone emulsifier | 1.00 - 10.00% |
| | **Aqueous phase: not more than 90%** | |
| 10 | Aqua (water) | 5.00 - 70.00% |
| 11 | Zinc salt (organic or inorganic) | 0.10 - 5.00% calculated as zinc |
| 12 | Dexpanthenol | 0.10 - 5.00% |
| 13 | Allantoin | 0.01 - 1.00% |
| 14 | Pharmaceutical glycerin | 0.10 - 10.00% |

The composition is in liquid form and consists of an aqueous phase and a silicone phase. It is commercially available in aerosol packaging and is intended for application in a spray form. There are two phases in the package: the aqueous phase and the silicone phase, after mixing which (the product should be shaken before application), a suspension is formed. An emulsifier (surfactant) causes a reduction in the surface tension between the two phases so that phase separation does not occur or does not occur too quickly - that is, at least for the duration of the application, it is a suspension.

The general composition of the above composition in aerosol packaging is shown below (the aerosol packaged product contains a spray and a pressurised carrier gas):

| | **Components** | % **by weight** |
|---|---|---|
| | **Silicone phase: not less than 10%** | |
| 1 | Silicone oil with a viscosity range of 6 cSt - 300,000 cSt The preferable viscosity range for silicone oil is 20 cSt - 100,000 cSt | 10.00 - 80.00% |
| 2 | Volatile silicone with viscosity in the range of 0.65 cSt - 1.0 cSt | 5.00 - 30.00% |
| 3 | Silicone elastomer | 1.00 - 60.00% |
| 4 | Vegetable oils | 5.00 - 30.00% |
| 5 | Silicone resin | 0.50-20.00% |
| 6 | Silicone rubber | 0.50-20.00% |
| 7 | Alkylated dimethicone | 0.10-30.00% |
| 8 | Silicone emulsifier | 0.50 - 10.00% |
| | **Aqueous phase: not more than 90%** | |
| 9 | Aqua | 5.00 - 70.00% |
| 10 | Zinc salt (organic or inorganic) | 0.10 - 5.00% calculated as zinc |
| 11 | Allantoin | 0.01-1.00% |
| 12 | Dexpanthenol | 0.1-5.00% |
| 13 | Pharmaceutical glycerin | 0.10-10.00% |
| | **Propellant:** | |
| 14 | Propane, butane, isobutane | 20.00 - 40.00 % |

After pressing the applicator, the valve opens and the gas pushes the charge outwards in the form of a fine stream. The aerosol packaging is a metal container with an applicator, enabling contactless, uniform application of a thin preparation layer, which is particularly important in the case of irritated and damaged skin. It is also worth noting that the spray form is very convenient and allows for quick and easy application of the preparation to a specific surface, which is especially important for older people.

Compliance is thus ensured (adherence to medical recommendations with particular emphasis on the systematic use of medicinal and supportive treatment products).

### Technological process:

The silicone phase components were combined separately and stirred until a homogeneous solution was obtained. The water phase components were combined separately and stirred until the individual components were completely dissolved.

Then both phases were combined and the whole was homogenized for several minutes. The obtained charge was poured into aerosol containers and the containers were filled with carrier gas.

The process was carried out at room temperature.

The present invention will become more comprehensible based on the exemplary formulations 1-17 presented below.

### Formulation 1:

| | **Component** | **% by weight** |
|---|---|---|
| 1 | Dimethicone (350 cSt) | 40.00% |
| 2 | Trisiloxane (1.0 cSt) | 15.00% |
| 3 | Dimethicone, Dimethicone Copolymer | 20.00% |
| 4 | Capryl methicone | 10.00% |
| 5 | Purified water | 12.50% |
| 6 | Zinc citrate | 2.50% |

### Composition in aerosol packaging:

| | **Component** | % **by weight** |
|---|---|---|
| 1 | Dimethicone (20 cSt) | 35.00% |
| 2 | Trisiloxane (1.0 cSt) | 10.00% |
| 3 | Dimethicone, Dimethicone Copolymer | 15.00% |
| 4 | Capryl methicone | 5.00% |
| 5 | Purified water | 12.50% |
| 6 | Zinc citrate | 2.50% |
| 8 | Propellant: mixture of propane, butane, isobutane | 20.00% |

### Formula 2:

| | **Component** | **% by weight** |
|---|---|---|
| 1 | Dimethicone (20 cSt) | 40.00% |
| 2 | Trisiloxane (1.0 cSt) | 15.00% |
| 3 | Dimethicone, Dimethicone Copolymer | 15.00% |
| 4 | Capryl methicone | 15.00% |
| 5 | Purified water | 14.00% |
| 6 | Zinc gluconate | 1.00% |

### Composition in aerosol packaging:

| | **Component** | **% by weight** |
|---|---|---|
| 1 | Dimethicone (20 cSt) | 35.00% |
| 2 | Trisiloxane (1.0 cSt) | 10.00% |
| 3 | Dimethicone, Dimethicone Copolymer | 10.00% |
| 4 | Capryl methicone | 10.00% |
| 5 | Purified water | 14.00% |
| 6 | Zinc gluconate | 1.00% |
| 7 | Propellant: carrier gas mixture: propane, butane, isobutane | 20.00% |

### Formulation 3:

| | **Component** | **% by weight** |
|---|---|---|
| 1 | Dimethicone (20 cSt) | 40.00% |
| 2 | Trisiloxane (1.0 cSt) | 15.00% |
| 3 | Dimethicone, Dimethicone Copolymer | 20.00% |
| 4 | Capryl methicone | 10.00% |
| 5 | Purified water | 13.00% |
| 6 | Zinc PCA | 2.00% |

### Composition in aerosol packaging:

| | **Component** | **% by weight** |
|---|---|---|
| 1 | Dimethicone (20 cSt) | 35.00% |
| 2 | Trisiloxane (1.0 cSt) | 10.00% |
| 3 | Dimethicone, Dimethicone Copolymer | 10.00% |
| 5 | Capryl methicone | 10.00% |
| 6 | Purified water | 13.00% |
| 7 | Zinc PCA | 2.00% |
| 8 | Propellant: carrier gas mixture: propane, butane, isobutane | 20.00% |

### Formulation 4:

| | **Component** | **% by weight** |
|---|---|---|
| 1 | Dimethicone (20 cSt) | 35.00% |
| 2 | Dimethicone (12,500 cSt) | 5.00% |
| 3 | Trisiloxane (1.0 cSt) | 15.00% |
| 3 | Dimethicone, Dimethicone Copolymer | 15.00% |
| 5 | Capryl methicone | 15.00% |

| | | |
|---|---|---|
| 6 | Purified water | 13.00% |
| 7 | Zinc PCA | 2.00% |

### Composition in aerosol packaging:

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Purified water | 13.00% |
| 7 | Zinc PCA | 2.00% |
| 8 | Propellant: carrier gas mixture: propane, butane, isobutane | 20.00% |

### Formulation 5:

| | **Component** | **% by weight** |
|---|---|---|
| 1 | Dimethicone (20 cSt) | 40.00% |
| 2 | Trisiloxane (1.0 cSt) | 15.00% |
| 3 | Dimethicone, Dimethicone Copolymer | 15.00% |
| 4 | Capryl methicone | 15.00% |
| 5 | Purified water | 12.50% |
| 6 | Zinc lactate | 2.50% |

### Composition in aerosol packaging:

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Purified water | 12.50% |
| 7 | Zinc lactate | 2.50% |
| 8 | Propellant: carrier gas mixture: propane, butane, isobutane | 20.00% |

### Formulation 6:

| | **Component** | **% by weight** |
|---|---|---|
| 1 | Dimethicone (20 cSt) | 35.00% |
| 2 | Dimethicone(12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Purified water | 12.50% |
| 7 | Zinc sulphate | 2.50% |

### Composition in aerosol packaging:

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Purified water | 12.50% |
| 7 | Zinc sulphate | 2.50% |
| 8 | Propellant: carrier gas mixture: propane, butane, isobutane | 20.00% |

### Formulation 7:

| | **Component** | **% by weight** |
|---|---|---|
| 1 | Dimethicone (20 cSt) | 35.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Purified water | 10.00% |
| 7 | Zinc PCA | 2.00% |
| 8 | Pharmaceutical glycerin | 3.00% |

### Composition in aerosol packaging:

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Purified water | 10.00% |
| 7 | Zinc PCA | 2.00% |
| 8 | Pharmaceutical glycerine | 3.00% |
| 9 | Propellant: carrier gas mixture: propane, butane, isobutane | 20.00% |

### Formulation 8:

| | **Component** | **% by weight** |
|---|---|---|
| 1 | Dimethicone (20 cSt) | 35.00% |
| 2 | Dimethicone (12,500 cSt) | 5.00% |
| 3 | Trisiloxane (1.0 cSt) | 15.00% |
| 4 | Dimethicone, Dimethicone Copolymer | 10.00% |
| 5 | Capryl methicone | 10.00% |
| 6 | Purified water | 12.00% |
| 7 | Zinc PCA | 2.00% |
| 8 | Dexpanthenol | 1.00% |

### Composition in aerosol packaging:

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane(1.0 cSt) | 10.00% |
| 4 | Dimethicone, Dimethicone Copolymer | 10.00% |
| 5 | Capryl methicone | 10.00% |
| 6 | Purified water | 12.00% |
| 7 | Zinc PCA | 2.00% |
| 8 | Dexpanthenol | 1.00% |
| 9 | Propellant: carrier gas mixture: propane, butane, isobutane | 20.00% |

### Formulation 9:

| | **Component** | **% by weight** |
|---|---|---|
| 1 | Dimethicone (20 cSt) | 35.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Purified water | 12.50% |
| 7 | Zinc PCA | 2.00% |
| 8 | Allantoin | 0.5% |

### Composition in aerosol packaging:

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Purified water | 12.50% |
| 7 | Zinc PCA | 2.00% |
| 8 | Allantoin | 0.5% |
| 9 | Propellant: carrier gas mixture: propane, butane, isobutane | 20.00% |

### Formulation 10

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Sweet almond oil | 5.00% |
| 7 | Purified water | 12.50% |
| 8 | Zinc PCA | 2.00% |
| 9 | Allantoin | 0.5% |

### Composition in aerosol packaging:

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Macadamia oil | 5.00% |
| 7 | Purified water | 12.50% |
| 8 | Zinc PCA | 2.00% |
| 9 | Allantoin | 0.5% |
| 10 | Propellant: carrier gas mixture: propane, butane, isobutane | 20.00% |

### Formulation 11

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Sweet almond oil | 5.00% |
| 7 | Purified water | 12.50% |
| 8 | Zinc PCA | 2.00% |
| 9 | Allantoin | 0.5% |

### Composition in aerosol packaging:

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Sweet almond oil | 5.00% |
| 7 | Purified water | 12.50% |
| 8 | Zinc PCA | 2.00% |
| 9 | Allantoin | 0.5% |
| 10 | Propellant: carrier gas mixture: propane, butane, isobutane | 20.00% |

### Formulation 12

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Moringa oil | 5.00% |
| 7 | Purified water | 12.50% |
| 8 | Zinc PCA | 2.00% |
| 9 | Allantoin | 0.5% |

### Composition in aerosol packaging:

| | **Component** | % **by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Moringa oil | 5.00% |
| 7 | Purified water | 12.50% |
| 8 | Zinc PCA | 2.00% |
| 9 | Allantoin | 0.5% |
| 10 | Propellant: carrier gas mixture: propane, butane, isobutane | 20.00% |

### Formulation 13

| | **Component** | **% by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 10.00% |
| 6 | Moringa oil | 5.00% |
| 7 | Caprylic/Capric Acid Triglycerides | 5.00% |
| 8 | Purified water | 12.50% |
| 9 | Zinc PCA | 2.00% |
| 10 | Allantoin | 0.5% |

### Composition in aerosol packaging:

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 10.00% |
| 4 | Dimethicone, Dimethicone Copolymer | 5.00% |
| 5 | Capryl methicone | 10.00% |
| 6 | Moringa oil | 5.00% |
| 7 | Caprylic/Capric Acid Triglycerides | 5.00% |
| 8 | Purified water | 12.50% |
| 9 | Zinc PCA | 2.00% |
| 10 | Allantoin | 0.5% |
| 11 | Propellant: carrier gas mixture: propane, butane, isobutane | 20.00% |

### Formulation 14

| | **Component** | % **by weight** |
|---|---|---|
| 1 | Dimethicone (20 cSt) | 30.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Capryl methicone | 15.00% |
| 6 | Linseed oil | 5.00% |
| 7 | Purified water | 12.50% |
| 8 | Zinc PCA | 2.00% |
| 9 | Allantoin | 0.5% |

### Composition in aerosol packaging:

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Capryl methicone | 10.00% |
| 6 | Linseed oil | 5.00% |
| 7 | Purified water | 12.50% |
| 8 | Zinc PCA | 2.00% |
| 9 | Allantoin | 0.5% |
| 10 | Propellant: carrier gas mixture: propane, butane, isobutane | 20.00% |

### Formulation 15

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Cetyl PEG/PPG-10/1 Dimethicone | 2.00% |
| 6 | Capryl methicone | 18.00% |
| 7 | Moringa oil | 5.00% |
| 8 | Purified water | 13.00% |
| 9 | Zinc PCA | 2.00% |

### Composition in aerosol packaging:

| | **Component** | **% by weight** |
|---|---|---|
| 1 | Dimethicone (20 cSt) | 20.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Cetyl PEG/PPG-10/1 Dimethicone | 2.00% |
| 6 | Capryl methicone | 13.00% |
| 7 | Moringa oil | 5.00% |
| 8 | Purified water | 13.00% |
| | Zinc PCA | 2.00% |
| 9 | Propellant: carrier gas mixture: propane, butane, isobutane | 20.00% |

### Formulation 16

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Cetyl PEG/PPG-10/1 Dimethicone | 2.00% |
| 6 | Capryl methicone | 13.00% |
| 7 | Moringa oil | 5.00% |
| 8 | Dimetikonol | 5.00% |
| 9 | Purified water | 13.00% |
| 10 | Zinc PCA | 2.00% |

### Composition in aerosol packaging:

| | **Component** | **% by weight** |
|---|---|---|
| 1 | Dimethicone (20 cSt) | 20.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Cetyl PEG/PPG-10/1 Dimethicone | 2.00% |
| 6 | Capryl methicone | 10.00% |
| 7 | Moringa oil | 5.00% |
| 8 | Dimethiconol | 3.00% |
| 9 | Purified water | 13.00% |
| 10 | Zinc PCA | 2.00% |
| 11 | Propellant: carrier gas mixture: propane, butane, isobutane | 20.00% |

### Formulation 17

| | **Component** | **% by weight** |
|---|---|---|
| 1 | Dimethicone (20 cSt) | 25.00% |
| 2 | Dimethicone (12,500 cSt) | 5.00% |
| 3 | Trisiloxane (1.0 cSt) | 15.00% |
| 4 | Dimethicone, Dimethicone Copolymer | 15.00% |
| 5 | Cetyl PEG/PPG-10/1 Dimethicone | 2.00% |
| 6 | Capryl methicone | 13.00% |
| 7 | Moringa oil | 5.00% |
| 8 | Trimethylsiloxysilicate | 5.00% |
| 9 | Purified water | 13.00% |
| 10 | Zinc PCA | 2.00% |

### Composition in aerosol packaging:

| | **Component** | **% by weight** |
|---|---|---|
| 1 | Dimethicone (20 cSt) | 20.00% |
| 2 | Dimethicone (12,500 cSt) | 5.00% |
| 3 | Trisiloxane (1.0 cSt) | 10.00% |
| 4 | Dimethicone, Dimethicone Copolymer | 10.00% |
| 5 | Cetyl PEG/PPG-10/1 Dimethicone | 2.00% |
| 6 | Capryl methicone | 10.00% |
| 7 | Moringa oil | 5.00% |
| 8 | Trimethylsiloxysilicate | 3.00% |
| 9 | Purified water | 13.00% |
| 10 | Zinc PCA | 2.00% |
| 11 | Propellant: carrier gas mixture: propane, butane, isobutane | 20.00% |

Alternatively, the composition may be in a semi-solid form.

The trial aimed to evaluate the efficacy of the pharmaceutical composition according to the invention (hereinafter referred to as the investigational product according to the nomenclature of clinical trials) for the prevention and alleviation of early skin reactions caused by irradiation in patients treated for cancer. The trial was a prospective, observational trial. The patients received a skin care composition according to the invention, specifically selected from the formulations 1.1-1.15 shown above.

Patients used the investigational product from the first day of irradiation until the onset of skin lesions in the form of erythema and possibly slight dry desquamation, which is the onset of a skin reaction of grade I severity according to the EORTC/RTOG scale. The primary endpoint was the indication of the percentage of patients who reached grade III of the reaction severity according to the RTOG/EORTC scale.

The secondary endpoints were an indication of the percentage of patients who reached grade II of the reaction severity according to the EORTC/RTOG scale, the time to the onset of skin damage of grade III in EORTC/RTOG scale and the time to healing of post-irradiation reaction of grade III in EORTC/RTOG scale. The general condition of the patients and their quality of life were also evaluated in this trial.

The trial included patients with diagnosed and histopathologically confirmed cancer of the head and neck region, qualified for the treatment with the intention of radical irradiation as a stand-alone method or in combination with chemotherapy and/or surgery, who gave informed consent to participate in the trial.

Exclusion criteria:
- Poor general condition,
- Disqualification from treatment with a radical approach,
- Eligibility for radical intent treatment with EGFR receptor-targeted drugs,
- Coexistence of dermatological diseases manifested by changes in the epidermis or dermis located in the irradiated volume,
- Lack of written consent from the patient.

The duration of each patient's participation in the trial was 11-12 weeks. During the course of the trial, in addition to the visit qualifying for participation in the program, patients had 4 or 5 follow-up visits, respectively: visit 1-day 14±3 days, visit 2-day 28±3 days, visit 3 - 42 day±3 days, visit 4-49 day±3 days, and a follow-up visit 30 days after completion of radiotherapy.

### Results:

### Time to the onset of the first reaction:

The trial showed that the mean time to the onset of the first-grade reaction is 21.0 ± 9.7 days, median 14 days, range from 11 to 45 days. Usually, the first reaction occurs even after about 10 hours from the first irradiation. It occurs in the form of redness (erythema) and is usually not permanent. This means that the use of the pharmaceutical composition according to the invention significantly prolonged the time to the onset of reaction 1.

### Patients' overall health general condition and quality of life

At the follow-up visit, the condition of patients was evaluated according to the ECOG scale shown below in Table 1 (Eastern Cooperative Oncology Group Performance Status Scale) that measures a cancer patient's overall health general condition and quality of life):

**Table 1**

| **Performance status** | **Definition** |
|---|---|
| 0 | normal fitness, ability to carry out daily activities independently |
| 1 | presence of disease symptoms, ability to walk and perform light work |
| 2 | ability to perform personal activities, inability to work, spends about half the day in bed |
| 3 | limited ability to perform personal activities, spends more than half the day in bed |
| 4 | secondary care needed, spends all day in bed |
| 5 | Dead |

The evaluation revealed, as shown in Table 2 below that as many as nearly 91% of the patients had normal performance (grade 0), 5.5% of the patients - grade 1 and 3.6% - grade 2. None of the patients had a performance status of grade 4 or 5.

**Table 2 - Patients' status according to the ECOG scale (numbers in brackets denote the number of trial participants who were diagnosed with a given performance grade):**

| | **ECOG status scale performance evaluation** | | | |
|---|---|---|---|---|
| **Visit** | **0** | **1** | **2** | **3** |
| **v0** | 86.0% (49) | 14.0% (8) | 0.0% (0) | 0.0% (0) |
| **v1** | 89.5% (51) | 10.5% (6) | 0.0% (0) | 0.0% (0) |
| **v2** | 86.0% (49) | 14.0% (8) | 0.0% (0) | 0.0% (0) |
| **v3** | 63.2% (36) | 36.8% (21) | 0.0% (0) | 0.0% (0) |
| **v4 (n=41)** | 56.1% (23) | 36.6% (15) | 4.9% (2) | 2.4% (1) |
| **v30 (n=55)** | 90.9% (50) | 5.5% (3) | 3.6% (2) | 0.0% (0) |

| | | | | |
|---|---|---|---|---|
| n - number of participants in the trial | | | | |

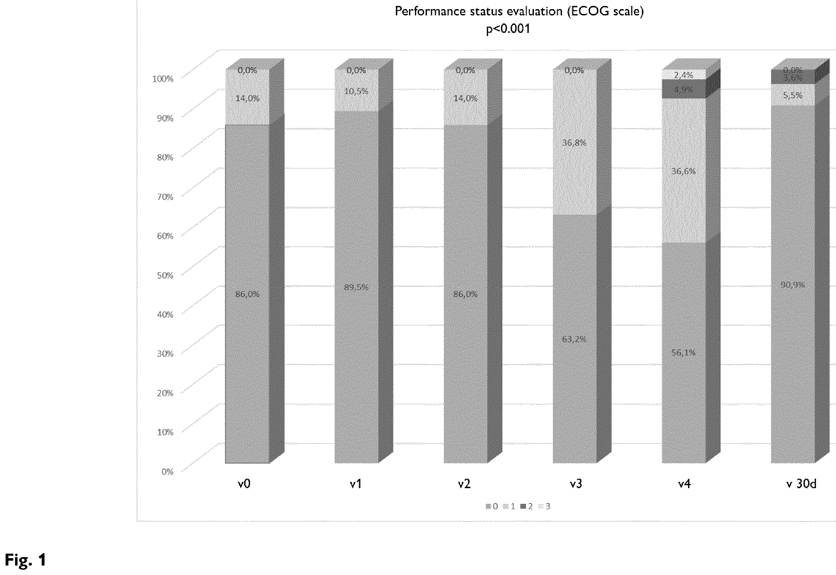

Fig. 1 shows that in the vast majority of patients (90.9%) the performance status after the end of the trial was evaluated as normal (grade 0), in 5.5% - grade 1 (presence of disease symptoms) and in 3.6% - grade 2 (limited ability to perform personal activities).

It can therefore be stated that thanks to the use of the product with silicone, the condition of the trial participants in most cases was normal, despite the applied therapy that usually significantly reduces the overall performance status of the patients.

It can be stated that the general condition and quality of life of patients undergoing radiotherapy, during which the pharmaceutical composition according to the invention was used to prevent the onset of severe post-irradiation reactions, have been evaluated very well. There were also statistically significant differences in the sensation of itching, burning and general well-being between visit 0 and the follow-up visit (30 days after completion of radiotherapy), as shown in Table 3 below. The VAS scale was used to measure these parameters. The scale contains 11 degrees of severity of a given sensation - from 0 to 10. The higher the score, the greater the feeling of negative symptoms.

**Table 3**

| | VAS v0 versus v 30 days | | |
|---|---|---|---|
| VAS | Therapy | Completion of therapy | Statistical significance p |
| Pain | 60.0 | 1.11 | 0.267 |
| Itching | 6.0 | 3.91 | <0.001 |
| Burning | 14.0 | 2.42 | 0.016 |
| General well-being | 275.0 | 2.02 | 0.044 |

The trial examined whether there was a statistically significant difference in the quality of life evaluation on the VAS scale during therapy versus after its completion. Statistically significant differences have been found for the variable: itching, burning, general well-being - here the obtained probability value p was lower than the adopted level of statistical significance. These results also confirm that the applied intervention has a positive effect on the quality of life of oncological patients.

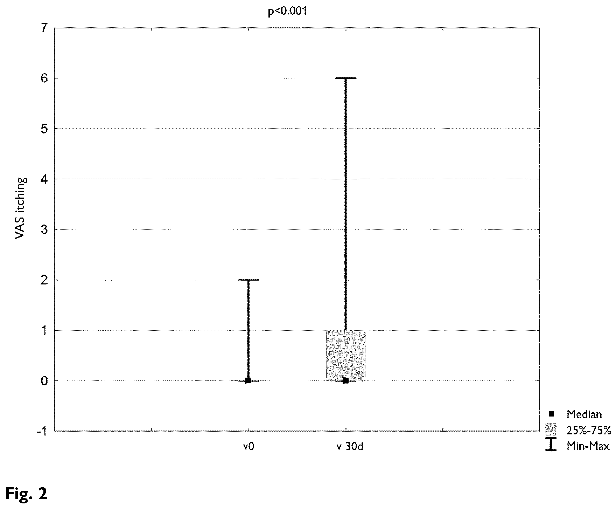

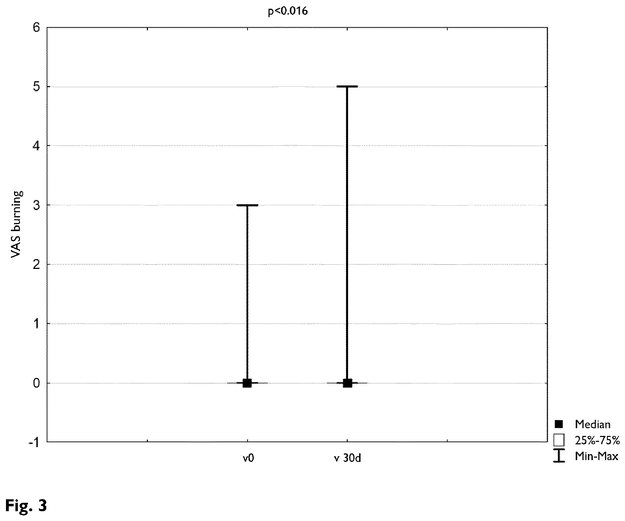

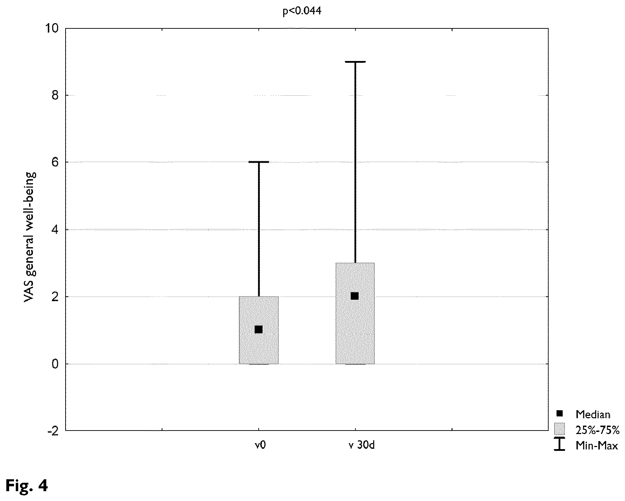

The applied product with silicone had a positive effect on the patients and reduced their unpleasant feelings accompanying radiotherapy. As can be seen from the results of figs. 1-4, the applied composition according to the invention had a beneficial effect on the patients and reduced their unpleasant feelings accompanying radiotherapy.

The use of the pharmaceutical composition according to the invention delays the time to the onset of the first reaction, furthermore improves the quality of life of patients and their general condition. The composition exhibits anti-oxidant and free radical-fighting properties. In the TEWL parameter test for the obtained compositions after 4 hours after application, a TEWL reduction of not less than 10% was obtained. Equipment tests of the TEWL parameter were performed under standardised environmental conditions, that is at the temperature 20-25°C and at relative humidity 40-60%, using a Tewameter TM HEX - a device equipped with sensors measuring humidity and temperature that allows the analysis of transepidermal water loss.

## Claims

1. Pharmaceutical composition for use in prevention and alleviation of early skin reactions caused by irradiation in patients treated for cancer consisting of an aqueous phase and a silicone phase, the aqueous phase comprises at least one water-soluble compound as a source of zinc ions and the content of the silicone phase in the composition is not less than 10% by weight, wherein the water-soluble compound being the source of zinc ions is preferably a zinc salt selected from organic or inorganic salts in the amount of 0.10 - 5.00% calculated as zinc and more preferably the organic salt of zinc is selected from the group comprising pyrrolidonecarboxylic acid zinc salt (zinc PCA), zinc lactate, zinc gluconate, zinc citrate and mixtures thereof.

2. The composition for use according to claim 1, **characterised in that** the silicone phase comprises silicon oil, wherein preferably the amount of silicon oil is in the range of 10.00 - 90.00% by weight.

3. The composition for use according to claim 2, **characterised in that** the silicone oil is selected from the group comprising dimethicones and mixtures thereof.

4. The composition for use according to claim 2 or 3, **characterised in that** the viscosity of silicone oil is in the range of 6 cSt - 300,000 cSt.

5. The composition for use according to claim 1, **characterised in that** the silicone phase comprises a silicone elastomer, wherein preferably the amount of the silicone elastomer is between 1.00 and 60.00% by weight.

6. The composition for use according to claim 5, **characterised in that** the silicone elastomer is selected from the group comprising dimethicone copolymer, dimethicone and vinyl dimethicone copolymer and mixtures thereof.

7. The composition for use according to claim 1, **characterised in that** the silicone phase comprises a silicone resin, wherein the silicone resin is preferably trimethylsiloxysilicate.

8. The composition for use according to any one of claims 1-7, **characterised in that** the silicone phase comprises a silicone rubber, wherein the silicone rubber is preferably dimethiconol.

9. The composition for use according to any one of claims 1-8, **characterised in that** the silicone phase comprises at least one alkylated dimethicone selected from the group comprising capryl methicone, cetyl dimethicone, stearyl dimethicone and mixtures thereof.

10. The composition for use according to any one of claims 1-9, **characterised in that** the silicone phase further comprises vegetable oil as a single oil or a mixture of oils, in a total amount of 1.00 - 30.00% by weight.

11. The composition for use according to any one of claims 1-10, **characterised in that** the silicone phase further comprises caprylic and capric acid triglycerides in a total amount of 1.00 - 30.00% by weight.

12. The composition for use according to any one of claims 1-11, **characterised in that** the silicone phase further comprises a volatile silicone, preferably the amount of volatile silicone is in the range of 5.00 - 30.00% by weight, preferably the viscosity of the volatile silicone is in the range of 0.65 cSt - 8.0 cSt, preferably the volatile silicone is selected from the group comprising trisiloxane, disiloxane, hexamethyldisiloxane, cyclotetrasiloxane, cyclopentasiloxane, cyclohexasiloxane and mixtures thereof.

13. The composition for use according to any one of claims 1-12, **characterised in that** the silicone phase comprises silicone oil alone or in combination with an ingredient selected from the group comprising silicone elastomer, silicone resin, silicone rubber, alkylated dimethicone and mixtures thereof, wherein preferably the silicone phase further comprises a silicone emulsifier.

14. The composition for use according to any one of claims 1-13, **characterised in that** the aqueous phase further comprises allantoin, and/or the aqueous phase further comprises dexpanthenol, and/or the aqueous phase further comprises at least one polyhydric alcohol selected from the group comprising propylene glycol, glycerin, xylitol, sorbitol and mixtures thereof.

15. The composition for use according to any one of claims 1-14, intended for application in spray form.
